# EUROPEAN PATENT APPLICATION

(11) **EP 4 238 557 A1**
(43) Date of publication of application: **06.09.2023**
(21) Application number: 22160079.4
(22) Date of filing: 03.03.2022
(51) Int. Cl.: A61K 31/00, A61K 31/122, A61K 31/195, A61K 31/196, A61K 31/20, A61K 31/202, A61P 1/00

(54) **GPR120 AGONISTS FOR THE TREATMENT OF INFLAMMATORY BOWEL DISEASE**

(71) Applicant: Dompe' Farmaceutici S.P.A., 20122 Milan (IT)
(72) Inventor: ALLEGRETTI, Marcello, 67100 L'Aquila (IT); ARAMINI, Andrea, 67100 L'Aquila (IT); BIANCHINI, Gianluca, 67100 L'Aquila (IT); D'ALESSIO, Silvia, 26825 Lodi (IT)
(74) Representative: Mauri, Elisabetta Maria Ester

(57) **Abstract**

The present invention relates to GPR120 agonists for use in the treatment of inflammatory bowel disease, in particular ulcerative colitis or Crohn's disease.

## Description

### FIELD OF THE INVENTION

The present invention relates to GPR120 agonists for use in the treatment of inflammatory bowel disease.

### STATE OF THE ART

Inflammatory bowel disease (IBD) identifies a group of chronic inflammatory conditions of the gastrointestinal tract, among which the most common ones are ulcerative colitis (UC) and Crohn's disease (CD). In ulcerative colitis inflammation is limited to the mucosal layer of the large intestine (colon) and the rectum, while in Crohn's disease it most commonly affects the ileum, but can affect any part of the gastro-intestinal tract in patchy distribution, even the mouth.

Both disorders share similar symptoms such as diarrhea, abdominal pain, rectal bleeding, and weight loss. However, despite the similarity among them, there are some other sympthoms that differ. For example, malnutrition is very common in Crohn's disease because the small intestine is responsible for the absorption of nutrients and it is the one affected, ulcerative colitis, instead, is associated with blood in stool and rectal bleeding, less common in Crohn's disease. More than 50% of people with CD suffer from folate and vitamin D deficiency, while more than 50% of people with UC suffer from iron deficiency. Inflammatory bowel disease affects men and women equally, it often begins in adolescence and approximately 25% of patients with IBDs are younger than 20 years. In some cases, it may occur also in adulthood (Journal of Medicine and Life Vol. 12, Issue 2, April-June 2019, pp. 113-122). Although etiology of inflammatory bowel disease remains largely unknown, it arises from an extremely complex interaction among genetic and environmental elements, dysregulated immune response and alterations of the microbiome, but none of these factors alone is sufficient to cause the disease. To diagnose this disorder, clinical, endoscopic, histologic, and radiological tests are used (Zhang Y. et al. World J Gastroenterol 2014; 20(1): 91-99).

Currently, therapy of inflammatory bowel disease is based on a stage-by-stage approach. For mild to moderate Crohn's disease, the first step of treatment is the use of an aminosalicylate, like 5-aminosalicylic acid (5-ASA) also commercially named Mesalamine. If this does not show efficacy, antibiotics, corticosteroids and immunosuppressive drugs are used, as last attempt before hospitalization.

For ulcerative colitis, the conventional treatment includes 5-aminosalicylic acid, corticosteroids, and purine analogues (azathioprine and mercaptopurine). If these are not effective, patients are treated with immunosuppressive drugs such as calicineurin inhibitors, tacrolimus and TNF-α inhibitors (Baumgart DC et al. The Lancet 2007; 369(9573):1641-57).

However, existing therapeutic strategies do not always lead to remission and may be related to numerous side effects. Therefore, for a substantial number of patients, this debilitating disorder remains far from being controlled in a satisfactory manner. As such, IBD remains associated with an unmet medical need for many patients (Gordon et al, Eur. J. Gastroenterol. Hepatol. 2015 (27), 804-812).

It is therefore strongly felt the need to develop new therapeutic approaches for the treatment of inflammatory bowel disease, with a higher efficacy and less side effects.

A number of studies have shown beneficial effects of ω-3 polyunsaturated fatty acids administered as adjunctive therapy in the prevention or treatment of ulcertaive colitis and Chron's disease. It has also been demonstrated that ω-3 fatty acids are substrate to the production of protectins, resolvins, and maresins, which may regulate and attenuate the inflammatory processes and lead to remission of IBD and, thus, could be considered as a new complementary approach to the treatment of these inflammatory conditions (Marton et al, International Journal of Molecular Science 2019, 20 (19), 4851). GPR120 is a member of the rhodopsin family of G protein-coupled receptors (GPRs) and has been shown to mediate some of the anti-inflammatory and insulin-sensitizing effects of ω-3 fatty acids ( Young Oh, Cell. 2010 September 3; 142(5): 687-698).

It has been shown that GPR120 is abundantly expressed in the human intestine, in endocrine L, K- or I-cells, where it acts as an exteroceptor for free fatty acids. These cells are present in the intestinal villi and face toward the intestinal lumen in contact with food (Furness, J. B. et al. Nat. Rev. Gastroenterol. Hepatol. 2013, 10, 729-740). Other studies have identified that, in the terminal ileum and proximal colon, not only endocrine but all epithelial cells lining the villi express GPR120 (Paulsen et al, PLoS ONE 2014, 9(2): e88227, doi:10.1371/journal.pone.0088227).

### SUMMARY OF THE INVENTION

As will be shown in the experimental section, the present inventors have surprisingly found that the administration of a GPR120 agonist compound is able to effectively prevent or treat inflammatory bowel disease.

The inventors have also found that this therapeutic effect is dependent on the topical activity of the molecule at the site of inflammation in the gastrointestinal tract and, in particular, in the ileum and colon.

Therefore, when a GPR120 agonist is administered orally, often sufficient concentrations at the target site are not reached, which result in poor efficacy.

The inventors have also identified a group particularly effective GPR120 agonists that, upon oral administration, are poorly absorbed and concentrate in the ileum and colon at high concentrations and are thus able to more effectively prevent or treat inflammation associated to inflammatory bowel disease.

Accordingly, a first object of the present invention is a GPR120 agonist for use in the prevention or treatment of inflammatory bowel disease in an individual.

A further object of the present invention is a pharmaceutical composition comprising a GPR120 agonist and at least one inert pharmaceutically acceptable excipient, for use in the prevention or treatment of inflammatory bowel disease in an individual.

A further object of the present invention is a method for the prevention or treatment of inflammatory bowel disease in an individual comprising administering to the individual an effective amount of a GPR120 agonist.

### BRIEF DESCRIPTION OF THE FIGURES

Figure 1 represents the % of body weight loss **(****Figure 1A****)** and the DAI score **(****Figure 1B**) over time in the experimental groups described in Example 1a). DSS I, II and III indicate the phases of the disease development (acute/chronic). The experiment is described in example 1b).
Figure 2 shows the colon length, measured after mice sacrifice at day 39, in the experimental groups described in Example 1a), as described in example 1c).
Figure 3 shows the pictures of the endoscopic assay described in Example 1d), for each of the experimental groups described in Example 1a).
Figure 4 shows the composite endoscopic score, described in Example 1d), for each of the experimental groups described in Example 1a).
Figure 5 shows the thickening of the colon score **(****Figure 5A****)** and the vascular pattern score **(Figure 5B)** as described in Example 1d), for each of the experimental groups described in Example 1a).
Figure 6 shows the visible fibrin score **(****Figure 6A****)** and the granularity of the mucosal surface score **(****Figure 6B****)** as described in Example 1d), for each of the experimental groups described in Example 1a).
Figure 7 shows the histological analyses, as described in Example 1e) for each of the experimental groups described in Example 1a). It shows the histological sections stained with Hematoxylin and Eosin.
Figure 8 is the mean histological score for each parameter of the Rachmilewitz score shown in the table of Example 1e), for each of the experimental groups described in Example 1a).
Figure 9 shows a schematic representation of the GPR120 signalling.
Figure 10 shows for each of the experimental groups described in Example 1a), the results of the western blotting of GPR120 and β-arrestin 2 **(****Figure 10B****)** and a densitometric analysis of the blots of the western blotting, performed using Image J, and the resulting ratio between co-immunoprecipitated β-arrestin 2 and GPR120 **(****Figure 10A****)** levels for each experimental group as described in Example 1f).
Figure 11 shows the results of the western blotting of GPR40 and β-arrestin 2 **(****Figure 11B**) and a densitometric analysis of the blots of the western blotting, performed using Image J, and the resulting ratio between co-immunoprecipitated β-arrestin 2 and GPR40 levels for each experimental group **(****Figure 11A****),** as described in Example 1f).

### DEFINITIONS

According to the present invention, the term "prevention" refers to administration to an individual to obtain partial or complete prevention of a disorder or pathological event, before this is established or occurs.

According to one embodiment of the invention, prevention is a complete prevention, wherein the disorder or pathological event is completely blocked.

According to an alternative embodiment of the invention, prevention is a partial prevention, wherein the onset or the development of the disorder or pathological event is delayed or reduced in severity.

According to the present invention, the term "treatment" refers to complete reversal or reduction of severity or progression of a disorder or pathological event, after this is established or occurs.

According to the present invention, the term "individual" refers to a human or an animal being, preferably to a human being.

According to the present invention, the term "GP120" identifies G protein-coupled receptor 120 (GPR120), also known as Free fatty acid receptor **4.**

According to the present invention, the term "GPR120 agonist" refers to a compound that binds to GPR120 and activates GPR120 signaling pathways. Many GPR 120 agonists are known in the art. The activation of GPR120 signaling pathways by the agonist are assessed by in vitro assays well known to the skilled person, such as, for example, the calcium mobilization assay (Hirasawa et al., Nature Medicine 2005, Vol. 11 (1), doi:10.1038/nm1168) or the Beta-arrestin recruitment assay (Oh et al. Cell 2010, 142, 687-698).

### DETAILED DESCRIPTION OF THE INVENTION

A first object of the present invention is a GPR120 agonist for use in the prevention or treatment of inflammatory bowel disease in an individual.

Preferably, said inflammatory bowel disease is ulcerative colitis or Crohn's disease. The GPR120 agonist for use according to the invention is preferably a compound selected from the group consisting of:
Epanova, which consists of omega-3 fatty acid mixture comprising eicosapentaenoic acid (EPA) and docosahexaenoic acid (DHA);
Alpha-linolenic acid;
SR-3: a mixture of alpha-linolenic and linoleic acids in a weight ratio of 1:4;
(+)-KDT-501;
PBI-4547;
and
a compound of formula (I) 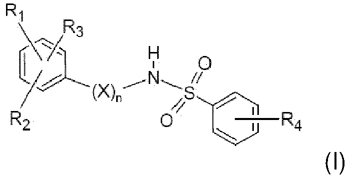 wherein
   R₁, R₂ and R₃ are independently selected from -H, -halogen, -CF₃, -OCH₃, -OH, phenyl, -OPh, -NO₂, -NH2, -N(CH₃)₂, linear or branched C₁-C₆ alkyl and a five-membered heterocycle ring.
   R₄ is -Y-C(O)OH, wherein Y is a straight chain C₄-C₁₄ hydrocarbon, saturated or unsaturated, preferably having from 6 to 8 carbon atoms,
   X is -CH₂ or -C(O),
   n is 0, 1 or 2.

Preferably, said compound of formula (I) is selected from:
a compound of formula (II): 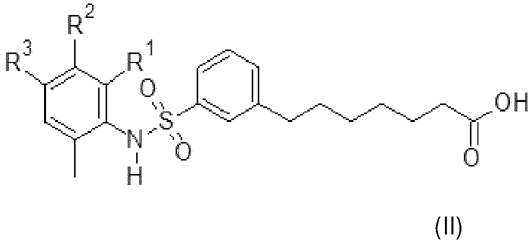 wherein:
   R¹ is CH₃,
   R² is H,
   R³ is selected from F, Cl and CF₃,
   or
   R¹ and R² are independently selected from substituted or unsubstituted phenyl or
   thiophen and H, provided that at least one of R¹ or R² is H, and
   R³ is F,
   wherein when said phenyl or thiophen is substituted, the substituent is preferably selected from Cl, F or CH₃, more preferably in position 2, 3 or 4 on the phenyl or in position 3, 4 or 5 on the thiophen; and
   the following compounds:
      7-(3-(N-(2,4,6-trimethylbenzyl)sulfamoyl)phenyl)heptanoic acid;
      7-(3-(N-(4-isopropyl-2,6-dimethylphenyl)sulfamoyl)phenyl)heptanoic acid;
      7-(3-(N-(4-(dimethylamino)-2,6-dimethylphenyl)sulfamoyl)phenyl)heptanoic acid;
      7-(3-(N-(4-bromo-2,6-dimethylphenyl)sulfamoyl)phenyl)heptanoic acid;
      7-(3-(N-(4-methoxy-2 ,6-dimethylphenyl)sulfamoyl)phenyl)heptanoic acid;
      6-{3-[(2,4,6-trimethylphenyl)sulfamoyl]phenyl}hexanoic acid;
      7-{3-[(4-fluoro-2,6-dimethylbenzoyl)sulfamoyl]phenyl}heptanoic acid;
      7-{4-[(4-fluoro-2,6-dimethylphenyl)sulfamoyl]phenyl}heptanoic acid;
      7-(3-(N-(4-hydroxy-2,6-dimethylphenyl)sulfamoyl)phenyl)heptanoic acid;
      7-(3-(N-(3,5-dimethyl-[1,1'-biphenyl]-4-yl)sulfamoyl)phenyl)heptanoic acid;
      7-(3-(N-(2,6-dimethyl-4-phenoxyphenyl)sulfamoyl)phenyl)heptanoic acid.

Preferably, said compound of formula (II) is selected from:
7-(3-(N-(4-fluoro-2,6-dimethylphenyl)sulfamoyl) phenyl)heptanoic acid;
7-(3-(N-(4-chloro-2,6-dimethylphenyl)sulfamoyl) phenyl)heptanoic acid;
7-(3-(N-(4-trifluoromethyl-2,6-dimethyl-phenyl)sulfamoyl)phenyl)heptanoic acid;
7-(3-(N-(6-fluoro-4-methyl-[1,1'-biphenyl]-3-yl)-2-sulfamoyl)phenyl)heptanoic acid;
7-(3-{[4-fluoro-2-methyl-5-(thiophen-2-yl)phenyl]sulfamoyl}phenyl)heptanoic acid, and
7-(3-(N-(5-fluoro-3-methyl-[1,1'-biphenyl]-3-yl)sulfamoyl)phenyl)heptanoic acid.

The administration of the GPR120 agonist for use according to the invention to the individual is in accordance with known methods.

Preferably, said administration is oral administration or rectal administration.

More preferably, the GPR120 agonist for use according to the invention is administered by oral administration.

As discussed above, the present inventors have found that the therapeutic effect of GPR120 agonists on inflammatory bowel disease is dependent on a topical activity of the molecule at the site of inflammation in the gastrointestinal tract. Therefore, when the GPR120 agonist is administered orally, sufficient delivery of active drug to the affected area is essential for therapeutic efficacy. At the same time, systemic availability of the compound is not useful for the therapeutic effect and should be reduced to a minimum to improve efficacy of the treatment and avoid any systemic side effects.

Thus, according to one embodiment, when the GPR120 agonist for use according to the invention is administered orally, preferably said use comprises administration of the GPR120 agonist in a pharmaceutical formulation designed to deliver sufficient amounts of the GPR120 agonist to the ileum and large instestine, which are the main sites of inflammatory lesions in IBD.

According to this embodiment, preferably, the GPR120 agonist in said pharmaceutical formulation is a GPR120 agonist which, when administered orally, is absorbed from the grastro-intestinal tract into the systemic circulation for more than 20 wt%, preferably more than 10 wt%, even more preferably more that 5 wt % of its total weight administered. According to this embodiment, more preferably, the GPR120 agonist in said pharmaceutical formulation is selected from:
Epanova: consisting of omega-3 fatty acid mixture comprising eicosapentaenoic acid (EPA) and docosahexaenoic acid (DHA);
Alpha-linolenic acid;
SR-3: a mixture of alpha-linolenic and linoleic acids in a weight ratio of 1:4; (+)-KDT-501;
PBI-4547;
7-(3-(N-(2,4,6-trimethylbenzyl)sulfamoyl)phenyl)heptanoic acid;
7-(3-(N-(4-isopropyl-2,6-dimethylphenyl)sulfamoyl)phenyl)heptanoic acid;
7-(3-(N-(4-(dimethylamino)-2,6-dimethylphenyl)sulfamoyl)phenyl)heptanoic acid;
7-(3-(N-(4-bromo-2,6-dimethylphenyl)sulfamoyl)phenyl)heptanoic acid;
7-(3-(N-(4-methoxy-2 ,6-dimethylphenyl)sulfamoyl)phenyl)heptanoic acid;
6-{3-[(2,4,6-trimethylphenyl)sulfamoyl]phenyl}hexanoic acid;
7-{3-[(4-fluoro-2,6-dimethylbenzoyl)sulfamoyl]phenyl}heptanoic acid;
7-{4-[(4-fluoro-2,6-dimethylphenyl)sulfamoyl]phenyl}heptanoic acid;
7-(3-(N-(4-hydroxy-2,6-dimethylphenyl)sulfamoyl)phenyl)heptanoicacid;
7-(3-(N-(3,5-dimethyl-[1 ,1'-biphenyl]-4-yl)sulfamoyl)phenyl)heptanoic acid; and
7-(3-(N-(2,6-dimethyl-4-phenoxyphenyl)sulfamoyl)phenyl)heptanoic acid.

According to this embodiment, preferably, said pharmaceutical formulation is a controlled release formulation. Prefarably, said controlled release formulation releases at least 70 weight %, more preferably at least 80 weight %, even more preferably at least 90 weight % of the active compound contained in the formulation, in the ileum and large intestine. More preferably, said controlled release formulation is a gastro-resistant formulation. As shown in the experimental section, the present inventors have also identified a number of GPR120 agonist compounds, having formula (II) described above, that, as demonstrated in Example 2, when administered orally, show a pharmacokinetic profile characterized by a very low systemic absorption and high concentrations reached in the lower gastrointestinal tract and are thus suitable for exerting a topical effect at the level of the areas of the gastrointestinal tract affected by IBD. These GPR120 agonists have high stability in the gastro and intestinal fluids and towards intestinal microsomes, and lastly, low permeability towards Caco-2 (immortalized cell line of human colorectal adenocarcinoma cells). Thanks to these characteristics, the GPR120 agonists realize a topical effect at the level of the mucosal layer of the ileum and large intestine, without the necessity of delivery with a controlled release formulation.

Thus, according to an alternative embodiment, when the GPR120 agonist for use according to the invention is administered orally, said GPR120 agonist for use according to the invention is preferably a compound of formula (II): 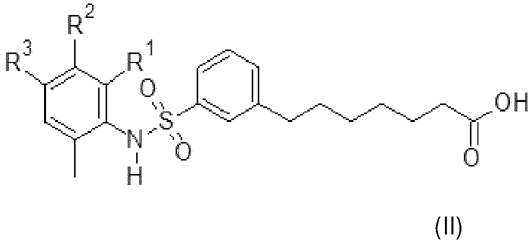 wherein:
R¹ is CH3,
R² is H,
R³ is selected from F, Cl and CF3,
or
R¹ and R² are independently selected from substituted or unsubstituted phenyl or thiophen and H, provided that at least one of R¹ or R² is H, and
R³ is F,
wherein when said phenyl or thiophen is substituted, the substituent is preferably selected from Cl, F or CH₃, more preferably in position 2, 3 or 4 on the phenyl or in position 3, 4 or 5 on the thiophen.

Preferably, said GPR120 agonist of formula (II) is selected from the group consisting of:
7-(3-(N-(4-fluoro-2,6-dimethylphenyl)sulfamoyl) phenyl)heptanoic acid;
7-(3-(N-(4-chloro-2,6-dimethylphenyl)sulfamoyl) phenyl)heptanoic acid;
7-(3-(N-(4-trifluoromethyl-2,6-dimethyl-phenyl)sulfamoyl)phenyl)heptanoic acid;
7-(3-(N-(6-fluoro-4-methyl-[1,1'-biphenyl]-3-yl)sulfamoyl)phenyl )heptanoic acid;
7-(3-{[4-fluoro-2-methyl-5-(thiophen-2-yl)phenyl]sulfamoyl}phenyl)heptanoic acid, and
7-(3-(N-(5-fluoro-3-methyl-[1,1'-biphenyl]-3-yl)sulfamoyl)phenyl)heptanoic acid.

Preferably, said GPR120 agonist of formula (II) for use according to the invention is administered in a pharmaceutical formulation that is not a controlled release formulation. Preferably, said GPR120 agonist of formula (II) for use according to the invention is administered in a pharmaceutical composition that is not a gastro-resistant formulation. A further object of the present invention is a pharmaceutical composition comprising a GPR120 agonist, as above defined, and at least one pharmaceutically acceptable excipient, for use in the prevention or treatment of inflammatory bowel disease in an individual, as above described.

Preferably, the pharmaceutical composition of the present invention is prepared in suitable dosage forms comprising an effective amount of the GPR120 agonist as above described, and at least one pharmaceutically acceptable excipient.

Preferably, the pharmaceutical composition of the present invention is for oral administration or rectal administration.

Preferably, said pharmaceutical composition for rectal administration is in form of a suppository, edema, gel or foam dosage form.

Preferably, said pharmaceutical composition for oral administration is in form of a granulate, fibres, microparticles, a tablet or a capsule.

In the present application, the wording "effective amount" means a dosage of a compound or composition sufficient to significantly achieve the desired clinical response.

The dosage and treatment regimens of the GPR120 agonist for use according to the invention for any particular patient will vary depending on a number of factors that are within the knowledge and expertise of the skilled person including, for example, the half life of the specific GPR120 agonist employed, the formulation and route of administration used, age, body weight, general health status, sex, and diet of the patient.

As described herein, the pharmaceutical composition of the present invention comprises a GPR120 agonist together with at least one pharmaceutically acceptable excipient, which, as used herein, is selected from solvents, diluents or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions.

The dosage forms of the pharmaceutical composition of the present invention can be prepared by techniques that are familiar to a pharmaceutical chemist, and comprise mixing, granulation, compression, dissolution, sterilization and the like. Further, the composition of the present invention may be suitably formulated using appropriate methods known in the art or by the method disclosed in Remington's Pharmaceutical Science (recent edition), Mack Publishing Company, Easton Pa.

The dosage forms can also contain other traditional ingredients such as: preservatives, stabilizers, surfactants, buffers, osmotic regulators, emulsifiers, sweeteners, colorants, flavourings and the like.

Preferably, the pharmaceutical composition for oral administration is in form of a controlled release formulation, more preferably a gatroresistant formulation.

More preferably, when the pharmaceutical composition is a controlled release formulation, more preferably a gastro-resistant formulation, the GPR120 agonist in said pharmaceutical formulation is a GPR120 agonist which, when administered orally, is absorbed from the grastro-intestinal tract into the systemic circulation for more than 20 wt%, preferably more than 10 wt%, even more preferably more that 5 wt % of its total weight administered..

More preferably, the GPR120 agonist in said controlled release formulation, more preferably gastro-resistant formulation, is selected from:
Epanova;
Alpha-linolenic acid;
SR-3;
(+)-KDT-501 ;
PBI-4547;
7-(3-(N-(2,4,6-trimethylbenzyl)sulfamoyl)phenyl)heptanoic acid;
7-(3-(N-(4-isopropyl-2,6-dimethylphenyl)sulfamoyl)phenyl)heptanoic acid;
7-(3-(N-(4-(dimethylamino)-2,6-dimethylphenyl)sulfamoyl)phenyl)heptanoic acid;
7-(3-(N-(4-bromo-2,6-dimethylphenyl)sulfamoyl)phenyl)heptanoic acid;
7-(3-(N-(4-methoxy-2 ,6-dimethylphenyl)sulfamoyl)phenyl)heptanoic acid;
6-{3-[(2,4,6-trimethylphenyl)sulfamoyl]phenyl}hexanoic acid;
7-{3-[(4-fluoro-2,6-dimethylbenzoyl)sulfamoyl]phenyl}heptanoic acid;
7-{4-[(4-fluoro-2,6-dimethylphenyl)sulfamoyl]phenyl}heptanoic acid;
7-(3-(N-( 4-hydroxy-2,6-dimethylphenyl)sulfamoyl)phenyl)heptanoic acid;
7-(3-(N-(3,5-dimethyl-[1,1'-biphenyl]-4-yl)sulfamoyl)phenyl)heptanoic acid;
7-(3-(N-(2,6-dimethyl-4-phenoxyphenyl)sulfamoyl)phenyl)heptanoic acid;

Preferably, said controlled release formulation, releases at least 70 weight%, more preferably at least 80 weight%, even more preferably at least 90 weight% of of the total weight of said GPR120 agonist contained in the formulation, in the ileum and large intestine.

Preferably, said controlled release formulation is a gastro-resistant formulation, which disintegrates at values of pH above 5.

Preferably said gastro-resistant formulation is in form of a coated table or of a soft or hard capsule.

Formulations having the above features are well known in the art.

For example, gastro-resistant tablets or capsules may be obtained by means of a coating with polymers that dissolve at pH above 5, such as methacrylate copolymers, for example the polymer commercialised under the trade names Eudragit L or S. For example, Eudragit L100-55 dissolves at a pH > 5.5 (duodenum targeting); Eudragit L100 dissolves at a pH 6-7 (jejunum); and Eudragit S100 at a pH > 7 (ileum and colon).

Also, gastro-resistant capsules, may be obtained by means of gastresistant shells, obtained by incorporation of the gastro-resistant polymers described above in the shell material used to form the capsules.

Furthermore, gastro-resistant capsules, may also be obtained by filling a hardshell capsule with pellets or minitablets coated with the gastro-resistant polymers described above.

For example, a tablet compostion suitable for the delivery of GPR120 agonist for use according of the invention by oral administration contains the following excipients: carboxypolymethylene 5-25% weight,
stearate magnesium 2-10% weight,
polyvinyl pyrrolydone (K30) 5-25%,
talcum 10-25% weight,
diethylphthalate 1-5% weight,
ester polymers of acrylic and methacrylic acid 10-30% weight.

The ester polymers of acrylic and methacrylic acid are used as dissolution profile modifier coating agent.

A further aspect of the present invention is a method for the prevention or treatment of inflammatory bowel disease in an individual comprising administering to the individual an effective amount of a GPR120 agonist, as above described.

### EXPERIMENTAL PART

### Statistical analyses.

In all experiments, statistical analyses were performed using GraphPad Prism 7 (GraphPad Software). Data are presented as means ±SD or ± SEM and differences were considered statistically significant when P < 0.05. For all the experiments an ANOVA multivariate analysis was performed accompanied by a post-hoc modification test.

### Example 1 - Therapeutic effect of DFL23806 in a mouse model of Dextran Sodium Sulfate-induced colitis

### a) Model set-up and treatment protocol

A mouse model of colitis induced by administration of Dextran Sodium Sulfate (DSS) (Wirtz S. et al. Nature Protocols; Volume: 2, Issue: 3 Pages: 541-546; 2007) was used to study the therapeutic effect of oral administration of DFL23806 on colitis.

In details, 8- to 12-week-old female C57/Bl6 mice were subjected to 3 oral cycles of 2.5% Dextran Sodium Sulfate (DSS) in drinking water (molecular mass, 40 kDa; MP Biomedicals), each cycle characterized by 7 days of DSS administration followed by 7 days of filter-purified water.

DSS administration induces clinical signs of disease as soon as 1 day post treatment, with changes in the expression of tight junction proteins and increased expression of pro-inflammatory cytokines. These modest initial effects are followed by increasingly drastic symptoms which are even more pronounced in the chronic model, as will be described below, including increased intestinal permeability, goblet cell depletion, epithelial erosion and ulceration, and severe bleeding. More in details, the current DSS-induced model of colitis is characterized by an acute and a chronic phase: acute clinical symptoms (diarrhea and/or grossly bloody stool) are associated with the presence of erosions and inflammation. More importantly, the earliest histologic changes are represented by the loss of the basal one-third of the crypt (day 3 after first DSS administration), which progressed with time to loss of the entire crypt resulting in erosions on day 5. The earliest changes are very focal and not associated with inflammation. Inflammation is a secondary phenomena and only become significant after erosions appear.

Animals treated with multiple cycles of DSS followed by cycles of water develop a chronic colitis with the following histologic features: areas of erosions and inflammation, inactivity, crypt distortion, florid epithelial proliferation and possible dysplasia. The clinical disease activity index correlates significantly with pathologic changes in both the acute and chronic phases of the disease (Chassaing, B, Current Protocols in Immunology 2014, 104(1), Cooper, H. S et al, Laboratory Investigation; a Journal of Technical Methods and Pathology 1993, 69(2), 238-249, D'Alessio, S et al, Journal of Clinical Investigation 2014, 124(9), 3863-3878)
From day 3 of DSS administration, the mice were divided in 4 experimental groups, each receiving the treatment specified below:
**Group 1: DSS (n=8)**
   No treatment, used as negative control.
**Group 2: DSS + Vehicle (n=8)**
   Treated with 20% v/v Dimethyl Sulfoxide (DMSO) and 80% v/v Phosphate Buffered Saline (PBS), 0.1 M, pH 8 (Vehicle), used as a negative control.
**Group 3: DSS** + **DFL 90 mg/kg (n=8)**
   Treated with DFL23806 dissolved in Vehicle, at the dosage of 90 mg/kg once a day.
**Group 4: DSS + 5-ASA 60 mg/kg (n=8)**
   Treated with 5-aminosalicylic acid (5-ASA) in Vehicle, at the dosage of 60 mg/Kg once a day, used as reference positive control.

Vehicle, DFL23806 and 5-ASA were administered daily, in the morning, by oral gavage, using plastic sterile feeding tubes 20ga x 30mm. All mice were sacrificed on day 39.

### b) Assessment of body weight loss and DAI (Disease Activity Index) score

A DAI score was established based on a scale that considers different parameters characterizing experimental colitis induction and progression (R S Walmsley et al. Gut 1998; 43:29-32).

Body weight, presence of gross blood in the feces, and stool consistency were recorded every 2 days. In details, the DAI was determined at each time point by scoring changes in weight loss (0 = none, 1 = 1 %-5%, 2 = 5%-10%, 3 = 10%-20%, 4 = >20%); stool consistency (0 = normal, 2 = loose, 4 = diarrhea); and rectal bleeding (0 = normal, 2 = occult bleeding, 4 = gross bleeding).

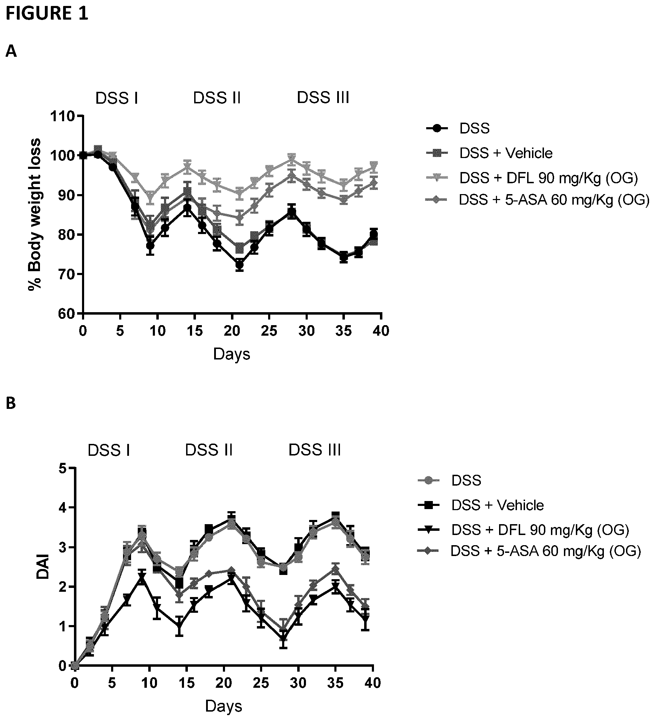

The results obtained show that the administration of DFL23806 shows a significant effect versus the controls (DSS and DSS+Vehicle groups), in terms of both reduced percentage of body weight loss **(****Figure 1A****),** and reduced DAI **(****Figure 1B****),** already at day 7. Interestingly, at certain time points DFL23806 was found to be even more effective than 5-ASA.

### c) Colon length measurement

Mice were sacrificed at day 39, colon were collected and colon lengths measured for each mouse, as additional parameter of intestinal inflammation.

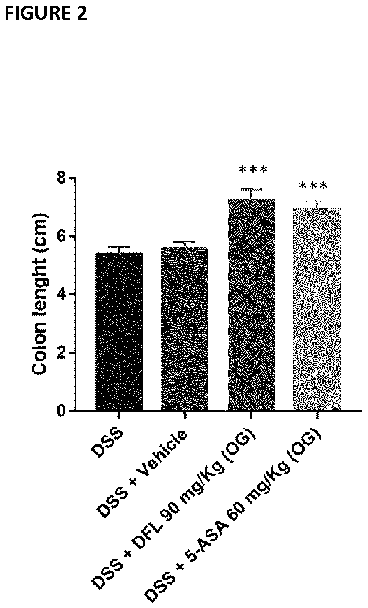

Results were confirmed by colon length, showing a significant inhibitory effect by DFL23806 and by 5-ASA, compared to the group DSS + vehicle **(****Figure 2****).**

### d) Endoscopic evaluation and score

The day of sacrifice (day 39), mice were anesthetized with 2% isoflurane and subjected to endoscopy, to confirm the inflammatory grade. The experimental endoscopy setup, denoted "Coloview system", consisted of a miniature endoscope (scope 1.9 mm outer diameter), a xenon light source, a triple chip camera, and an air pump (all from Karl Storz, Tuttlingen, Germany) to achieve regulated inflation of the mouse colon. The endoscopic procedure was viewed on a colour monitor and a modified murine endoscopic index score of colitis severity was assigned on the basis of colon translucency (0-3 points), granular features of the mucosa (0-3 points), morphology of the vascular pattern (0-4 points), and the presence of fibrin (0-4 points), as previously described by Dr. D'Alessio (D'Alessio, S. et al. J. Clin. Invest. 124, 3863-3878; 2014) leading to a cumulative score between 0 (no signs of inflammation) and 16 (endoscopic signs of very severe inflammation).

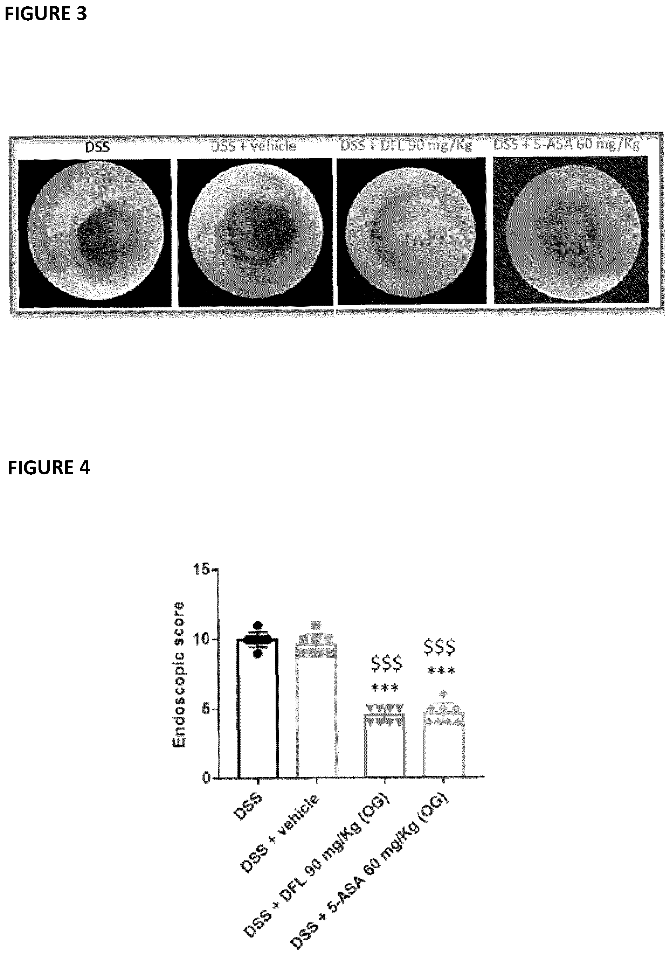

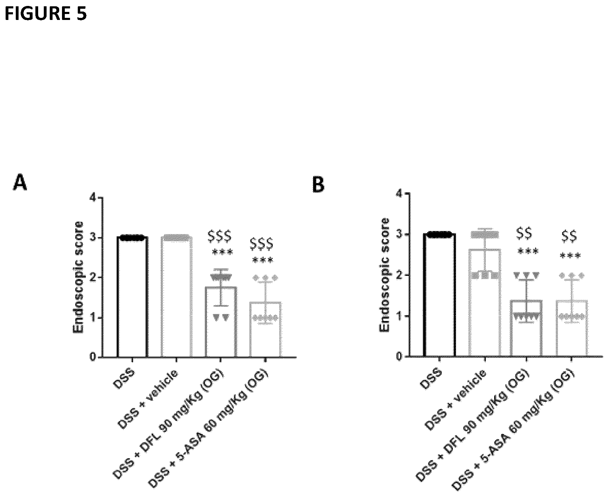

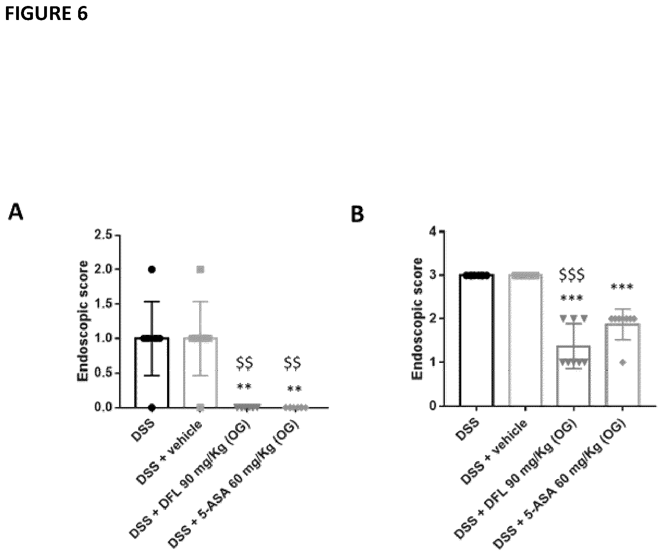

The composite endoscopic score obtained showed that DFL23806 and 5-ASA were able to significantly inhibit chronic experimental colitis compared to the controls (DSS and DSS+Vehicle groups) **(****Figure 3 and** 4) in a comparable manner. This was observed not only in terms of composite endoscopic score **(****Figure 4****),** but also for colon thickness **(****Figure 5A****),** vascular pattern **(Figure 5B),** visible fibrin **(****Figure 6A****),** and mucosal surface granularity **(****Figure 6B****).** Increased mucosal granularity represents edema and small erosions. Edema of the lamina propria is characterized by clusters of neutrophils and capillaries. These data indicate that promoting resolution of inflammation through oral administration of selective GPR120 agonist such as DFL23806, may help reducing neutrophil infiltration and mucosal edema.

### e) Histological evaluation and score

Histological analysis has been performed by an expert pathologist, in a blinded fashion, to grade the intestinal inflammation. More in details, colons of colitic mice in the various experimental groups have been evaluated for histological analyses using 2-µM paraffin-embedded sections, stained with hematoxylin (Dako) and eosin (Diapath). A blinded pathologist evaluated the degree of inflammatory cell infiltration and mucosal damage, using the RACHMILEWITZ score **(Table 1)** (Rachmilewitz, D. *etal. Gastroenterology***122*,*** 1428-1441; 2002).

This scoring system takes into account five histological parameters: ulceration, extent of ulceration, flogosis, extent of flogosis and fibrosis in the whole colon section, as shown in Table 1, without distinguishing into proximal and distal colon. For each parameter, a score between 0 and 4 is attributed as shown in Table 1. In the present case, since in the DSS-induced model of chronic colitis (Rachmilewitz, D. et al. Gastroenterology 122, 1428-1441; 2002) fibrosis does not develop, this parameter was not evaluated.

**Table 1. Rachmilewitz score**

| | | |
|---|---|---|
| Ulceration | 0 | No ulceration |
| | 1 | Involvement of the mucosa |
| | 2 | Involvement of the mucosa + submucosa |
| | 3 | involvement of the muscularis propria |
| | 4 | Involvement of the entire wall |
| Extension ulceration | 0 | No ulceration |
| | 1 | Punctate |
| | 2 | Minimal |
| | 3 | Moderate |
| | 4 | Widespread |
| Flogosis | 0 | Absent |
| | 1 | Minimal |
| | 2 | Slight |
| | 3 | Moderate |
| | 4 | Severe |
| Extension flogosis | 0 | Absent |
| | 1 | Mucosa |
| | 2 | Mucosa + submucosa |
| | 3 | Mucosa + submucosa + muscular wall penetration |
| | 4 | Entire wall |
| Fibrosis | 0 | Absent |
| | 1 | Mucosa |
| | 2 | Mucosa + submucosa |
| | 3 | Mucosa + submucosa + muscular wall penetration |
| | 4 | Entire wall |

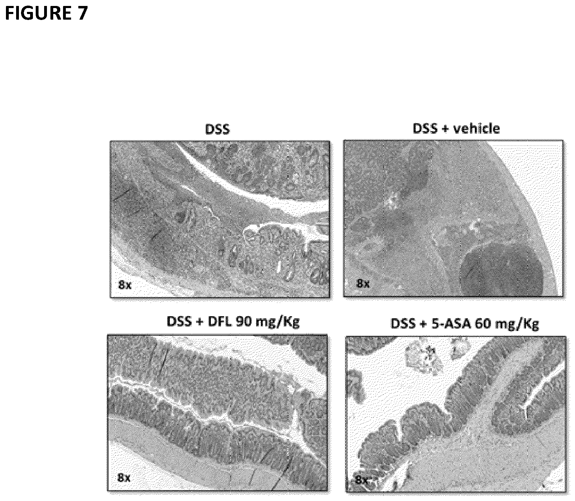

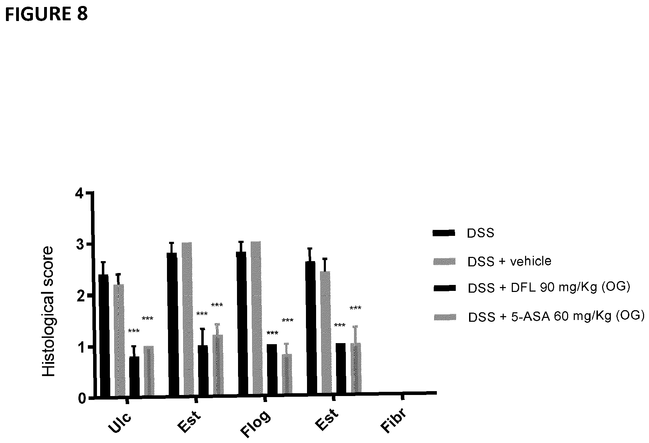

Representative histological images in **Figure 7** showed deep ulcerations, with crypt loss and massive inflammatory infiltrate in the DSS and DSS + Vehicle groups. Furthermore, results confirmed that both DFL and 5-ASA significantly reduced the histological score for ulceration, extent of ulceration, flogosis and extent of flogosis, when compared with DSS + Vehicle **(****Figure 8**), in a comparable manner.

### f) Evaluation of DFL binding to GPR120 and GPR40

The efficiency of DFL in activating the GPR120 and/or GPR40 receptors was tested.

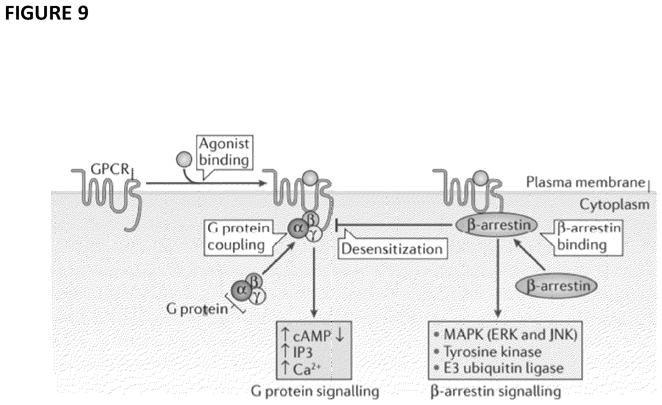

Agonist-induced GPR120 can engage multiple signalling pathways to regulate distinct physiological outcomes. After ligand binding, β-arrestins, such as β-arrestin 2 can associate with the cytoplasmic domains of GPR120 and couple the receptor to specific downstream signalling pathways **(****Figure 9**). For this reason, we verified the agonist efficiency in activating GPR120 and GPR40 by immunoprecipitation. Colon lysates (-900 µg proteins) from 6 mice of each experimental group were pooled, precleared with protein A/G plus-agarose (Santa Cruz) and then incubated with the antibody used for immunoprecipitation (anti-GPR120 or anti-GPR40, Abcam) at 4°C for 16 h, followed by incubation with protein A/G plus-agarose for 5 h. The agarose beads were then collected by centrifugation, washed four times with lysis buffer, and heated to 95°C for 5 min after adding Laemmli buffer. The resulting immunoprecipitates were separated by SDS-PAGE and probed with the anti-β-arrestin 2 antibody (Santa Cruz).

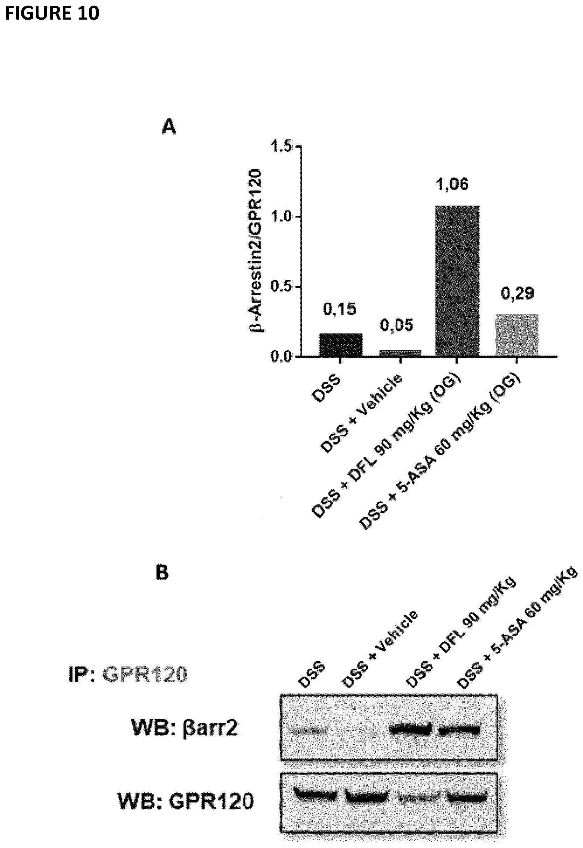

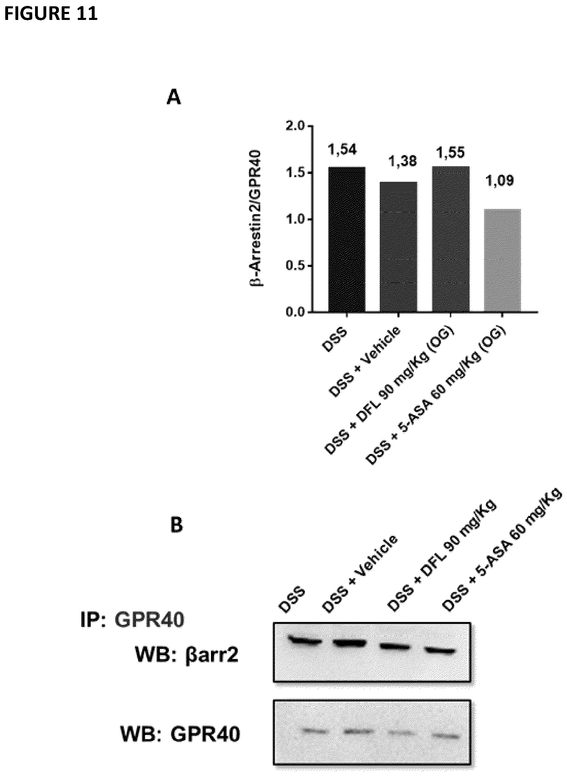

Upon western blotting, a densitometric analysis of the blots was performed using Image J. **Figure 10** **and** **11** show the ratio between co-immunoprecipitated β-arrestin 2 and GPR120 **(****Figure 10****)** or GPR40 levels **(****Figure 11****)** and the results of the blotting.

Results show that at day 39, DFL efficiently activates GPR120; in fact, the association between GPR120 and β-arrestin 2 in the DSS + DFL group is higher compared to control groups or to DSS + 5-ASA 60 mg/Kg group **(****Figure 10****).**

Upon immunoprecipitation with an anti-GPR40 antibody, results showed no increase of GPR40 and β-arrestin 2 association in the DSS + DFL group or in the DSS + 5-ASA group **(****Figure 11****),** compared to mice treated only with DSS or DSS + vehicle. This confirms that DFL is a GPR120 selective agonist.

### Example 2 - Evaluation of pharmacokinetics of GPR120 agonists after oral administration

The purpose of this study was to evaluate plasma and tissue exposure of the following compounds after a single oral administration to mice:
7-(3-(N-(4-fluoro-2,6-dimethylphenyl)sulfamoyl)phenyl)heptanoic acid (indicated below as DFL23806),
7-(3-(N-(4-chloro-2,6-dimethylphenyl)sulfamoyl)phenyl)heptanoic acid (indicated below as DFL23914)
7-(3-(N-(2,6-dimethyl-4-(trifluoromethyl)phenyl)sulfamoyl)phenyl)heptanoic acid (indicated below as DFL23922 )
7 -(3-(N-(6-fluoro-4-methyl-[1,1'-biphenyl]-3-yl)sulfamoyl)phenyl)heptanoic acid (indicated below as DFL23916)
7 -(3-(N-(5-fluoro-3-methyl-[1,1'-biphenyl]-2-yl)sulfamoyl)phenyl)heptanoic acid (indicated below as DFL23917)
7-(3-{[4-fluoro-2-methyl-5-(thiophen-2-yl)phenyl]sulfamoyl}phenyl)heptanoic acid (indicated below as DFL24102)

For each of the above compounds, nine male CD1 mice aged approximately 6/7 weeks at the beginning of the treatment period, were treated orally, by gastric gavage, with 55 mg/kg of the tested compound (adjusted for purity) dissolved in a vehicle consisting in 20% v/v Dimethyl Sulfoxide (DMSO) and 80% v/v Phosphate Buffered Saline (PBS), 0.1 M, pH 8.

A retro-orbital, composite blood sampling at pre-dose or after dosing at 30 min, 1, 2, 6, 8 and 24 hours (three mice/time point) was carried out for the plasma pharmacokinetic assessment. Blood samples were collected in heparinized collection tubes. Samples were taken immediately onto ice and kept cool until centrifugation (10000g for 3 minutes at about +4 °C). About 50 µL of plasma were stored in a freezer at -80 °C pending analysis.

All the animal procedures (including housing, health monitoring, restrain, dosing, etc) and ethical revision were performed according to the current Italian legislation (Legislative Decree March 4th, 2014 n. 26) enforcing the 2010/63/UE Directive on the protection of animals used for biomedical research.

After samples collection, bioanalysis for concentrations of the test compounds were done using HPLC-MS/MS methods and pharmacokinetic analysis of the compounds in plasma and tissues was performed according to standard non-compartmental approach using Watson system (v 7.6, Thermo Fisher Scientific Waltham, MA, USA) on mean concentration data.

The pharmacokinetic parameters obtained for each of the compounds tested are summarized in the following table:

**Table 2. Pharmacokinetic parameters after oral administration in the mouse**

| **Code** | **Dose_PO (mg/kg)** | **T1/2 (h)** | **Tmax (h)** | **Cmax (ng/mL)** | **AUC_last (ng/mL^{∗}h)** | **AUC_inf (ng/mL^{∗}h)** |
|---|---|---|---|---|---|---|
| DFL23806 | 55.0 | 1.7 | 2 | 62.7 | 262 | 305 |
| DFL23916 | 15.0 | 2.2 | 0.4 | 198 | 480 | 571 |
| DFL23914 | 10.0 | 1.2 | 2 | 45 | 189 | 230 |
| DFL23922 | 15.0 | 1.5 | 1.4 | 123 | 201 | 217 |
| DFL23917 | 15.0 | 1.3 | 0.5 | 135 | 378 | 402 |
| DFL24102 | 15.0 | 1.9 | 1 | 113 | 278 | 302 |

### Cmax values of DFL23806 in plasma, stomach, ileum and colon after single oral 55 mg/kg to male CD1 mice

| | Cmax plasma | Cmax stomach | Cmax ileum | Cmax colon |
|---|---|---|---|---|
| DFL23806 at 2h post dose | 62.7 ng/mL | 165000 ng/mL | 412 ng/mL | 190 ng/mL |

As can be seen from the table, for all the compounds tested, the concentrations in the investigated tissues were higher than the corresponding ones measured in plasma. The highest concentrations were measured in the stomach, followed by ileum and large intestine (colon).

None of the compounds was recovered in the urine in the time intervals 0-2, 2-8 and 8-24 hours and the amount recovered in feces was negligible, less than 0.01 % of the dose. These results demonstrate that the compounds tested concentrate in the tissues of the gastro-intestinal tract, thus exerting their activity locally.

## Claims

1. A GPR120 agonist for use in the prevention or treatment of inflammatory bowel disease in an individual.

2. A GPR120 agonist for use as claimed in claim 1, wherein said inflammatory bowel disease is ulcerative colitis or Crohn's disease.

3. A GPR120 agonist for use as claimed in claims 1 or 2, selected from:
- Epanova;
- Alpha-linolenic acid;
- SR-3;
- (+)-KDT-501;
- PBI-4547;
and
- a compound of formula (I) 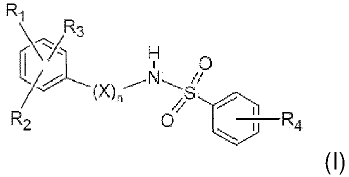 wherein
R₁, R₂ and R₃ are independently selected from -H, -halogen, -CF₃, -OCH₃, -OH, phenyl, -OPh, -NO₂, -NH₂, -N(CH₃)₂, linear or branched C₁-C₆ alkyl, and a five-membered heterocycle ring.
R₄ is -Y-C(O)OH, wherein Y is a straight chain C₄-C₁₄ hydrocarbon, saturated or unsaturated, preferably having from 6 to 8 carbon atoms,
X is -CH₂ or -C(O),
n is 0, 1 or 2.

4. A GPR120 agonist for use as claimed in claims 1 to 3, wherein said compound of formula (I) is selected from:
a compound of formula (II): 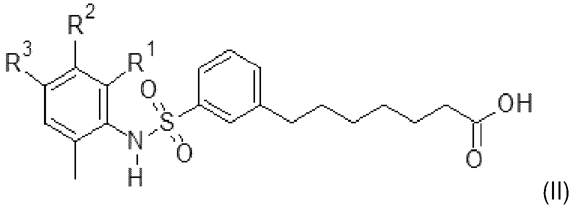 wherein:
R¹ is CH₃,
R² is H,
R³ is selected from F, Cl and CF₃,
or
R¹ and R² are independently selected from substituted or unsubstituted phenyl or
thiophen and H, provided that at least one of R¹ or R² is H, and
R³ is F,
wherein when said phenyl or thiophen is substituted, the substituent is preferably selected from Cl, F or CH₃, more preferably in position 2, 3 or 4 on the phenyl or in position 3, 4 or 5 on the thiophen; and
the following compounds:
7-(3-(N-(2,4,6-trimethylbenzyl)sulfamoyl)phenyl)heptanoic acid;
7-(3-(N-(4-isopropyl-2,6-dimethylphenyl)sulfamoyl)phenyl)heptanoic acid;
7-(3-(N-(4-(dimethylamino)-2,6-dimethylphenyl)sulfamoyl)phenyl)heptanoic acid;
7-(3-(N-(4-bromo-2,6-dimethylphenyl)sulfamoyl)phenyl)heptanoic acid;
7-(3-(N-(4-methoxy-2 ,6-dimethylphenyl)sulfamoyl)phenyl)heptanoic acid;
6-{3-[(2,4,6-trimethylphenyl)sulfamoyl]phenyl}hexanoic acid;
7-{3-[(4-fluoro-2,6-dimethylbenzoyl)sulfamoyl]phenyl}heptanoic acid;
7-{4-[ (4-fluoro-2,6-dimethylphenyl)sulfamoyl]phenyl}heptanoic acid;
7-(3-(N-(4-hydroxy-2,6-dimethylphenyl)sulfamoyl)phenyl)heptanoic acid;
7-(3-(N-(3,5-dimethyl-[1,1'-biphenyl]-4-yl)sulfamoyl)phenyl)heptanoic acid;
7-(3-(N-(2,6-dimethyl-4-phenoxyphenyl)sulfamoyl)phenyl)heptanoic acid.

5. A GPR120 agonist for use as claimed in claim 4, wherein said compound of formula (II) is selected from:
7-(3-(N-(4-fluoro-2,6-dimethylphenyl)sulfamoyl) phenyl)heptanoic acid;
7-(3-(N-(4-chloro-2,6-dimethylphenyl)sulfamoyl) phenyl)heptanoic acid;
7-(3-(N-(4-trifluoromethyl-2,6-dimethyl-phenyl)sulfamoyl)phenyl)heptanoic acid;
7-(3-(N-(6-fluoro-4-methyl-[1,1'-biphenyl]-3-yl)-2-sulfamoyl)phenyl)heptanoic acid,
7-(3-{[4-fluoro-2-methyl-5-(thiophen-2-yl)phenyl]sulfamoyl}phenyl)heptanoic acid, and
7-(3-(N-(5-fluoro-3-methyl-[1,1'-biphenyl]-3-yl)sulfamoyl)phenyl)heptanoic acid.

6. A GPR120 agonist for use as claimed in claims 1 to 5, wherein said GPR120 agonist is administered orally.

7. A GPR120 agonist for use as claimed in claim 6, wherein said GPR120 agonist is administered in a controlled release formulation, which releases at least 70 weight %, more preferably at least 80 weight %, even more preferably at least 90 weight % of the active compound contained in the formulation, in the ileum and large intestine.

8. A GPR120 agonist for use as claimed in claim 7, wherein said GPR120 agonist is selected from:
Epanova:
Alpha-linolenic acid;
SR-3;
(+)-KDT-501;
PBI-4547;
7-(3-(N-(2,4,6-trimethylbenzyl)sulfamoyl)phenyl)heptanoic acid;
7-(3-(N-(4-isopropyl-2,6-dimethylphenyl)sulfamoyl)phenyl)heptanoic acid;
7-(3-(N-(4-(dimethylamino)-2,6-dimethylphenyl)sulfamoyl)phenyl)heptanoic acid;
7-(3-(N-(4-bromo-2,6-dimethylphenyl)sulfamoyl)phenyl)heptanoic acid;
7-(3-(N-(4-methoxy-2 ,6-dimethylphenyl)sulfamoyl)phenyl)heptanoic acid;
6-{3-[(2,4,6-trimethylphenyl)sulfamoyl]phenyl}hexanoic acid;
7-{3-[(4-fluoro-2,6-dimethylbenzoyl)sulfamoyl]phenyl}heptanoic acid;
7-{4-[(4-fluoro-2,6-dimethylphenyl)sulfamoyl]phenyl}heptanoic acid;
7-(3-(N-(4-hydroxy-2,6-dimethylphenyl)sulfamoyl)phenyl)heptanoic acid;
7-(3-(N-(3,5-dimethyl-[1,1'-biphenyl]-4-yl)sulfamoyl)phenyl)heptanoic acid;
7-(3-(N-(2,6-dimethyl-4-phenoxyphenyl)sulfamoyl)phenyl)heptanoic acid;

9. A GPR120 agonist for use as claimed in claim 6, wherein said GPR120 agonist is a compound of formula (II): 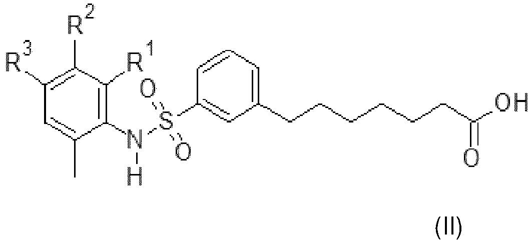 wherein:
R¹ is CH₃,
R² is H,
R³ is selected from F, Cl and CF₃,
or
R¹ and R² are independently selected from substituted or unsubstituted phenyl or
thiophen and H, provided that at least one of R¹ or R² is H, and
R³ is F,
wherein when said phenyl or thiophen is substituted, the substituent is preferably selected from Cl, F or CH₃, more preferably in position 2, 3 or 4 on the phenyl or in
position 3, 4 or 5 on the thiophen.

10. A GPR120 agonist for use as claimed in claim 9, wherein said GPR120 agonist of formula (II) is selected from the group consisting of:
7-(3-(N-(4-fluoro-2,6-dimethylphenyl)sulfamoyl) phenyl)heptanoic acid;
7-(3-(N-(4-chloro-2,6-dimethylphenyl)sulfamoyl) phenyl)heptanoic acid;
7-(3-(N-(4-trifluoromethyl-2,6-dimethyl-phenyl)sulfamoyl)phenyl)heptanoic acid;
7-(3-(N-(6-fluoro-4-methyl-[1,1'-biphenyl]-3-yl)sulfamoyl)phenyl )heptanoic acid;
7-(3-{[4-fluoro-2-methyl-5-(thiophen-2-yl)phenyl]sulfamoyl}phenyl)heptanoic acid
and
7-(3-(N-(5-fluoro-3-methyl-[1,1'-biphenyl]-3-yl)sulfamoyl)phenyl)heptanoic acid.

11. A GPR120 agonist for use as claimed in claim 9 or 10, wherein said GPR120 agonist of formula (II) is administered in a pharmaceutical composition that is not a controlled release formulation and/or gastro-resistant formulation.

12. A pharmaceutical composition comprising a GPR120 agonist of claims 1 to 11, and at least one inert pharmaceutically acceptable excipient, for use in the prevention or treatment of inflammatory bowel disease in an individual.

13. A pharmaceutical composition for use as claimed in claim 12, for oral administration.

14. A pharmaceutical composition for use as claimed in claim 13, wherein said composition is a controlled release formulation, which releases at least 70 weight%, more preferably at least 80 weight%, even more preferably at least 90 weight% of the total weight of said GPR120 agonist contained in the formulation, in the ileum and large intestine.

15. A pharmaceutical composition for use as claimed in claim 14, wherein said GPR120 agonist is selected from:
Epanova;
Alpha-linolenic acid;
SR-3;
(+)-KDT-501;
PBI-4547;
7-(3-(N-(2,4,6-trimethylbenzyl)sulfamoyl)phenyl)heptanoic acid;
7-(3-(N-(4-isopropyl-2,6-dimethylphenyl)sulfamoyl)phenyl)heptanoic acid;
7-(3-(N-(4-(dimethylamino)-2,6-dimethylphenyl)sulfamoyl)phenyl)heptanoic acid;
7-(3-(N-(4-bromo-2,6-dimethylphenyl)sulfamoyl)phenyl)heptanoic acid;
7-(3-(N-(4-methoxy-2 ,6-dimethylphenyl)sulfamoyl)phenyl)heptanoic acid;
6-{3-[(2,4,6-trimethylphenyl)sulfamoyl]phenyl}hexanoic acid;
7-{3-[(4-fluoro-2,6-dimethylbenzoyl)sulfamoyl]phenyl}heptanoic acid;
7-{4-[(4-fluoro-2,6-dimethylphenyl)sulfamoyl]phenyl}heptanoic acid;
7-(3-(N-(4-hydroxy-2,6-dimethylphenyl)sulfamoyl)phenyl)heptanoic acid;
7-(3-(N-(3,5-dimethyl-[1,1'-biphenyl]-4-yl)sulfamoyl)phenyl)heptanoic acid;
7-(3-(N-(2,6-dimethyl-4-phenoxyphenyl)sulfamoyl)phenyl)heptanoic acid.
